# EUROPEAN PATENT SPECIFICATION

(11) **EP 1 002 093 B1**
(45) Date of publication and mention of the grant of the patent: **06.04.2005**
(21) Application number: 98942007.0
(22) Date of filing: 06.08.1998
(51) Int. Cl.: C12N 15/44, C07K 14/08, C07K 19/00, C12N 15/62, C12N 15/70, C12N 1/21, A61K 39/145, C07K 16/10, A61K 39/42, G01N 33/569

(54) **PREPARATION AND USE OF RECOMBINANT INFLUENZA A VIRUS M2 CONSTRUCTS AND VACCINES**
HERSTELLUNG UND VERWENDUNG VON REKOMBINANTEN INFLUENZA A VIRUS M2 KONSTRUKTIONEN UND IMPSTOFFE
PREPARATION ET UTILISATION DE CONSTRUCTIONS DE LA PROTEINE M2 RECOMBINANTE DU VIRUS GRIPPAL A ET VACCINS CORRESPONDANTS

(30) Priority: 06.08.1997 US 906930
(43) Date of publication of application: 24.05.2000
(73) Proprietor: Centers for Disease Control & Prevention, Atlanta, GA 30333 (US)
(72) Inventor: FRACE, A., Michael, Atlanta, GA 30307 (US); KLIMOV, Alexander, I., Atlanta, GA 30345 (US); KATZ, Jacqueline, M., Atlanta, GA 30307 (US)
(74) Representative: Grund, Martin, Dr.
(86) International application number: PCT/US1998/016379
(87) International publication number: WO 1999/028478

(56) References cited:
- WO-A-93/03173
- GRAMBAS S ET AL: "Influence of amantadine resistance mutations on the pH regulatory function of the M2 protein of influenza A viruses." VIROLOGY, (1992 DEC) 191 (2) 541-9. JOURNAL CODE: XEA. ISSN: 0042-6822., XP002085466 United States
- HOLSINGER L J ET AL: "Influenza A virus M2 ion channel protein: a structure-function analysis." JOURNAL OF VIROLOGY, (1994 MAR) 68 (3) 1551-63. JOURNAL CODE: KCV. ISSN: 0022-538X., XP002085467 United States
- WANG C ET AL: "Activation of the M2 ion channel of influenza virus: a role for the transmembrane domain histidine residue." BIOPHYSICAL JOURNAL, (1995 OCT) 69 (4) 1363-71. JOURNAL CODE: A5S. ISSN: 0006-3495., XP002085468 United States
- WANG C ET AL: "Direct measurement of the influenza A virus M2 protein ion channel activity in mammalian cells." VIROLOGY, (1994 NOV 15) 205 (1) 133-40. JOURNAL CODE: XEA. ISSN: 0042-6822., XP002085469 United States

## Description

### BACKGROUND OF THE INVENTION

### Field of the Invention

The present invention relates to the field of vaccines against influenza A virus and the constructs useful in their production.

### Description of the Related Art

The prior art illustrates the current strategy for control of influenza by yearly vaccination with whole-virus or subunit vaccines. The currently-licensed vaccines are designed to stimulate neutralizing antibodies against hemagglutinin (HA) and/or neuraminidase (NA), the major surface antigens of the influenza virus. However, due to frequent and unpredictable structural variation of HA and NA, influenza vaccines must be seasonally customized to circulating virus strains, a process which is deficient in providing protective immunity against all but closely matched viral strains.

There is a need for a vaccine subunit component capable of inducing broader, more cross-reactive immunity to type A influenza viruses. One such component may be M2, a structurally conserved influenza A viral surface protein (Slepushkin *et al*., 1995; Ito *et al.,* 1991). The DNA sequences of the M2 genes of numerous influenza A viruses are known (Ito *et al*., 1991). M2 is thought to provide an obligatory transmembrane proton flux for viral replication (Sugrue *et al*., 1990; Clamper *et al*., 1992b; Grambas and Hay, 1992). As a membrane transport protein, M2 functions as an open pore which conducts cations in a nonselective manner (Tosteson *et al*., 1994; Shimbo *et al*., 1996). This conductance is thought to permeabilize host cells expressing recombinant M2 and may explain difficulties that others have had in achieving high levels of recombinant M2 expression in prokaryotic as well as eukaryotic systems (Guinea and Carrasco, 1996; Black *et al*., 1993).

Antibody to M2 has been shown to restrict influenza virus replication in cell culture and in infected mice (Zebedee and Lamb (1988) and Treanor *et al.,* (1990). Full length M2 expressed in baculovirus has been shown to raise serum titers and stimulate T-cell responses in immunized animals (Katz, *et al.,* 1996). Further, vaccination of mice with recombinant full-length M2 has been shown to enhance viral clearance from infected lungs and to provide protection from lethal challenge with heterologous influenza A virus (Slepushkin *et al*., 1995).

Since M2 is not expressed to any extent in virions (Zebedee & Lamb, 1988), the current whole virus or split-product influenza vaccine contains only minimal amounts of M2. To be useful as a component of a vaccine, M2 must be expressed and purified as a recombinant product. However, expression of full-length M2 has been shown to be detrimental to cell culture in prokaryotic and eukaryotic expression systems (Guinea and Carrasco, 1996; Black *et al*., 1993). To date, expression of sufficient quantities of recombinant M2 for use in experimental studies can only be accomplished by culturing eukaryotic host cells in the presence of the irreversible M2 inhibitor, amantadine.

Wholly apart from the challenges in expression of recombinant M2, the hydrophobic nature of full-length M2 compromises the yield and purity of M2 preparations and necessitates the use of detergents or other agents to maintain M2 in a soluble form. Certain such solubilizing agents are not desirable constituents of vaccine formulations. The present invention solves this shortcoming in the prior art by providing a modified M2 protein with reduced hydrophobicity and concomitantly enhanced solubility characteristics relative to full-length M2.

### SUMMARY OF THE INVENTION

The present invention solves the problems of the prior art approaches to recombinant M2 production by providing new recombinant forms of M2 whose structure has been modified to allow simple prokaryotic expression as a soluble, readily purified variant protein that retains antigenic and immunogenic properties. A preferred embodiment of the present invention provides a recombinant construct in which at least the entire portion of the transmembrane domain has been deleted. Alternatively, residues within the transmembrane domain may simply be altered, for example by substitution of hydrophilic or neutral amino acid residues for hydrophobic residues, in such a way as to (a) enhance expression of the protein in prokaryotic and/or eukaryotic systems relative to the native protein and/or (b) render the modified M2 protein more soluble in aqueous solutions relative to the native M2 protein. The terms M2 polypeptide and M2 protein are used interchangeably herein. The present invention further provides vaccines comprised of these new recombinant forms of M2 and to methods of prevention and treatment of influenza A virus infections.

The foregoing merely summarizes certain aspects of the invention and is not intended, nor should it be construed as limiting the invention in any manner. All patent applications, patents, and other publications recited herein are hereby incorporated by reference in their entirety.

### BRIEF DESCRIPTION OF THE DRAWINGS

Fig. 1 is a schematic diagram of M2 and deletion constructs sM2 and ssM2;
Fig. 2 represents growth curves showing the time course of cell replication and expression of full-length and modified M2 polypeptides;
Fig. 3 (A) is a coomassie-stained SDS-PAGE gel; and
Fig. 3 (B) is a Western blot of fusion proteins containing modified M2 polypeptides.

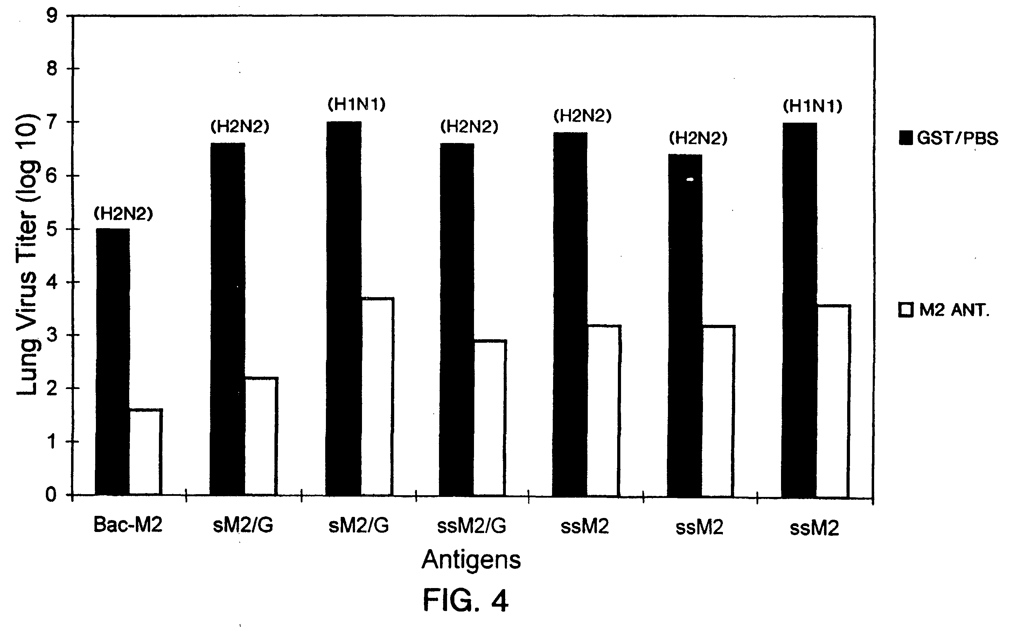

Fig. 4 displays the results of vaccination with M2 constructs as described in Example 7.

### DETAILED DESCRIPTION OF THE INVENTION

In a first aspect, the present invention provides for a modified M2 protein comprising the native M2 protein in which at least the hydrophobic, transmembrane region is deleted or substituted with neutral or hydrophilic residues. We have discovered that such a modified M2 protein is more soluble in aqueous solution relative to its native M2 protein counterpart and in a form suitable for high-yield expression and purification. Moreover, as demonstrated herein, modified M2 proteins according to this aspect of the invention are both immunogenic and immunoprotective, making them suitable for use as vaccines.

For the purposes of this invention, the transmembrane region of M2 is defined generally as that portion of the M2 polypeptide which spans all or part of the lipid bilayer of the influenza A virus surface. Residues 26-43 of the native M2 of the A/Aichi/2/68 (H3N2) virus correspond to the transmembrane region, although the modified M2 of the present invention can be constructed to correspond to the native M2 protein of any strain. Those skilled in the art will appreciate that comparable regions of other influenza A viruses and newly emerging influenza A viruses will correspond to this general description of a transmembrane region and the present invention contemplates removal or alteration of sufficient residues within the transmembrane region to render said region functionally inactive and, preferably, to reduce overall hydrophobicity, thereby allowing for efficient expression and purification of modified M2 polypeptides following culture in prokaryotic and eukaryotic hosts.

As mentioned, the modified M2 proteins according to the invention manifest enhanced expression in host organisms compared to the expression level of the native M2 protein. Although the invention is not limited by any theory, enhanced expression may arise due to inactivation (or significantly diminution) of the ion channel activity of the modified M2 polypeptide, thereby decreasing the polypeptide's toxicity to the expressing host organism. Preferably, modified M2 proteins of the invention are capable of being expressed in a host organism at levels of 5-50 mg/l or at levels sufficient to produce a visible band on coomassie stained gel.

In a preferred embodiment, the modified M2 protein according to this aspect of the invention comprises a sequence of amino acids identical to a native M2 protein in which the transmembrane region and from zero to twelve amino acid residues adjacent to the transmembrane region on its C-terminus side have been deleted. By this is meant that the modified M2 protein comprises the portion of a native M2 protein on the N-termirial side of the transmembrane region fused to a portion of the native M2 protein from the C-terminal side of the transmembrane region. In another preferred embodiment, the modified M2 protein comprises a sequence of amino acids identical to the native M2 protein of the A/Aichi/2/68 (H3N2) virus in which residues 26 through anywhere from 43 to 55 have been deleted. In other words, this embodiment comprises the N-terminal 25 amino acid sequence fused at its C-terminus to the N-terminal amino acid of the C-terminal portion of the native M2 protein, wherein the C-terminal portion begins (at its N-terminal end) at one of amino acid numbers 44-55 of the native M2 protein. In a more preferred embodiment, the modified M2 protein comprises a sequence of amino acids identical to the native M2 protein of the A/Aichi/2/68 (H3N2) virus in which residues 26-43 have been deleted. In another more preferred embodiment, the modified M2 protein comprises a sequence of amino acids identical to the native M2 protein of the A/Aichi/2/68 (H3N2) virus in which residues 26-55 have been deleted.

In another embodiment, the deleted residues of the native M2 protein are replaced with one or more neutral or hydrophilic amino acids. In this embodiment, the number of amino acid residues in the modified M2 protein is less than or equal to the number in the native M2 protein. The deleted residues are preferably replaced with from one to six neutral or hydrophilic amino acid residues. In another preferred embodiment, the neutral or hydrophilic residues in the foregoing embodiments are glycine.

In another preferred embodiment, the modified M2 protein according to this aspect of the invention comprises a sequence of amino acids identical to the native protein in which all of the amino acid residues of the transmembrane region and from zero to twelve amino acid residues adjacent to the transmembrane region on its C-terminus side have been substituted with neutral or hydrophilic amino acids. In this embodiment, the modified M2 protein has the same number of amino acid residues as the native M2 protein. The number of amino acid substitutions and the type of substitution are sufficient to yield a protein having a higher solubility in aqueous solution than the native protein and generally increased expression in host organisms. There are numerous such proteins according to this embodiment, and it is but a routine matter for one of ordinary skill in the art to substitute one or more of the known hydrophilic and/or neutral amino acids into the transmembrane region and/or the region adjacent to it on the C-terminal side to obtain a modified M2 protein according to this embodiment of the invention. Preferably the modified M2 protein according to this aspect of the invention has, except for the substituted amino acids, a sequence identical to the native M2 of the A/Aichi/2/68 (H3N2) virus.

In another preferred embodiment, the modified M2 protein according to this aspect of the invention comprises any one of the previously recited embodiments in the form of a fusion protein. In one embodiment, the modified M2 protein is fused to a polypeptide that renders the fusion construct more easily purified than the modified M2 protein alone. In a preferred embodiment, the modified M2 protein is fused to the glutathione S-transferase (GST) (*e.g.*, from *Schistosoma japonicum*). Alternatively or additionally, the modified M2 protein can be fused to a signal peptide so as to direct secretion of the polypeptide from the expressing host cell. It is but a routine matter for those skilled in the art to identify, make, and use other fusion proteins according to the invention employing a wide variety of polypeptides.

Those skilled in the art will recognize that the modified M2 polypeptides of the present invention can be produced by any one of a variety of recombinant methods. The basic steps in the recombinant production of modified M2 polypeptides include:
a) construction of a synthetic or semi-synthetic DNA encoding the modified M2 polypeptide,
b) integrating said DNA into an expression vector in a manner suitable for the expression of the modified M2 polypeptide either alone or as a fusion protein,
c) transforming an appropriate eukaryotic or prokaryotic host cell with said expression vector,
d) culturing said transformed or transfected host cell, and
e) recovering and purifying the recombinantly produced modified M2 polypeptides.

For recombinant expression, the modified M2 coding sequence may be wholly synthetic, semi-synthetic or the result of modification of the native M2 gene sequence.

In another aspect, the invention provides synthetic genes, the *in vitro* or *in vivo* transcription and translation of which will result in the production of modified M2 polypeptides. Such genes are derived from the gene sequence of the native M2 protein and suitably modified to encode the particular modified M2 protein of which expression is desired. Genes according to this aspect may be constructed by techniques well known in the art. Owing to the natural degeneracy of the genetic code, the skilled artisan will recognize that a sizable yet definite number of DNA sequences may be constructed that encode modified M2 polypeptides. The gene encoding the modified M2 polypeptides may be created by synthetic methodology. Such methodology of synthetic gene construction is well known in the art. The DNA sequence corresponding to the modified M2 polypeptide genes can be generated using conventional DNA synthesizing apparatus such as the Applied Biosystems Model 380A or 380B DNA synthesizers (commercially available from Applied Biosystems, Inc., 850 Lincoln Center Drive, Foster City, CA 94404).

One of ordinary skill in the art will recognize that the nucleotide sequence for the M2 gene from other isolates of influenza type A viruses will be very similar, though not necessarily identical, to the M2 gene sequence for A/Aichi/2/68 (H3N2). The teachings herein are readily applicable to closely related sequences from other influenza A type viruses, using techniques well established in the art. Accordingly, this invention contemplates variants of other M2 genes in which, as here, the transmembrane and/or hydrophobic regions have been deleted. Hybridization and wash conditions and protocols for obtaining sequences of a desired degree of homology are standard and well known to those skilled in the art. See, *e.g., Current Protocols in Molecular Biology,* vol. 1, unit 2.10 (John Wiley & Sons, Inc. 1997). These techniques can be employed on a routine basis to isolate homologous DNA molecules according to this aspect of the invention. Routine adjustment of the hybridization and wash conditions enable artisan of ordinary skill to obtain DNA of virtually any desired degree of homology. Nucleic acids according to this embodiment can be obtained using the protocol set forth in Chapter 5, Table 3 of *Nucleic Acid Hybridization: A Practical Approach* (Hames & Higgins, Eds., IRL Press, Washington D.C., 1985).

### Vector Construction Generally

Construction of suitable vectors containing the desired coding and control sequences employ standard ligation techniques. Isolated plasmids or DNA fragments are cleaved, tailored, and religated in the form desired to form the plasmids required.

To effect the translation of the modified M2 polypeptides, one inserts the engineered modified M2 DNA coding sequence in any of a plethora of appropriate recombinant DNA expression vectors through the use of appropriate restriction endonucleases. A synthetic modified M2 coding sequence is designed to possess restriction endonuclease cleavage sites at either end of the transcript to facilitate isolation from and integration into these expression and amplification and expression plasmids. The coding sequence may be readily modified by the use of synthetic linkers to facilitate the incorporation of this sequence into the desired cloning vectors by techniques well known in the art. The particular endonucleases employed will be dictated by the restriction endonuclease cleavage pattern of the parent expression vector to be employed. The choice of restriction sites are chosen so as to properly orient the modified M2 coding sequence with control sequences to achieve proper in-frame reading and expression of the modified M2 polypeptide genes.

In general, plasmid vectors containing promoters and control sequences which are derived from species compatible with the host cell are used with these hosts. The vector ordinarily carries a replication site as well as marker sequences which are capable of providing phenotypic selection in transformed cells. For example, *E. coli* is typically transformed using pBR322, a plasmid derived from an *E. coli* species (Bolivar, *et al.,* 1977). pBR322 contains genes for ampicillin and tetracycline resistance and thus provides easy means for identifying transformed cells. The pBR322 plasmid, or other microbial plasmid must also contain or be modified to contain promoters and other control elements commonly used in recombinant DNA construction.

The modified M2 polypeptide coding sequence must be positioned so as to be in proper reading frame with the promoter and ribosome binding site of the expression vector, both of which are functional in the host cell in which the modified M2 polypeptide is to be expressed. In the preferred practice of the invention, the promoter-operator region is placed in the same sequential orientation with respect to the ATG start codon of DNA sequence encoding the modified M2 polypeptide as the promoter-operator occupies with respect to the ATG-start codon of the gene from which it was derived. Synthetic or modified promoter-operator regions such as the tac promoter are well known in the art. When employing such synthetic or modified promoter-operator regions they should be oriented with respect to the ATG start codon of the modified M2 polypeptide coding sequence as directed by their creators.

### Prokaryotic Expression

In general, prokaryotes are used for cloning of DNA sequences in constructing the vectors useful in the invention. For example, *E. coli* K12 strain 294 (ATCC No. 31446) is particularly useful. Other microbial strains which may be used include *E. coli* B and *E. coli* X1776 (ATCC No. 31537), *E. coli* W3110 (prototrophic, ATCC No. 27325), bacilli such as *Bacillus subtilis,* and other enterobacteriaceae such as *Salmonella typhimurium* or *Serratia marcescans,* and various pseudomonas species may be used. Promoters suitable for use with prokaryotic hosts include the b-lactamase (vector pGX2907 [ATCC 39344] contains the replicon and b-lactamase gene) and lactose promoter systems (Chang *et al.,* 1978; Goeddel *et al*., 1979), alkaline phosphatase, the tryptophan (trp) promoter system (vector PATH1 [ATCC 37695] is designed to facilitate expression of an open reading frame as a trpE fusion protein under control of the trp promoter) and hybrid promoters such as the tac promoter (isolatable from plasmid pDR540 ATCC-37282). However, other functional bacterial promoters, whose nucleotide sequences are generally known, enable one of skill in the art to ligate them to DNA encoding modified M2 polypeptides using linkers or adapters to supply any required restriction sites. Promoters for use in bacterial systems also will contain a Shine-Dalgarno sequence operably linked to the DNA encoding modified M2 polypeptides. These examples are illustrative rather than limiting.

### Fusion Proteins

The modified M2 polypeptides may be made either by direct expression or as fusion protein comprising the modified M2 polypeptide followed by enzymatic or chemical cleavage. It is often observed in the production of certain peptides in recombinant systems that expression as a fusion protein prolongs the lifespan and/or increases the yield of the desired peptide. A variety of peptidases (*e.g.*, trypsin) which cleave a polypeptide at specific sites or digest the peptides from the amino or carboxy termini (*e.g.*, diaminopeptidase) of the peptide chain are known. Furthermore, particular chemicals (*e.g.*, cyanogen bromide) will cleave a polypeptide chain at specific sites. The skilled artisan will appreciate the modifications necessary to the amino acid sequence (and synthetic or semi-synthetic coding sequence if recombinant means are employed) to incorporate site-specific internal cleavage sites. See e.g., Carter P. (1990).

Therefore, it may be desirable to fuse the coding sequence of a particular modified M2 polypeptide in-frame to a larger gene coding sequence resulting in the production of a fusion protein.

### Eukaryotic Expression

The modified M2 polypeptides can also be recombinantly produced in eukaryotic expression systems.

### Eukaryotic Signal Peptides

An advantage of eukaryotic expression systems is that it is possible to obtain a secreted protein product. If such a result is desired, it is necessary to modify the coding sequence of the modified M2 polypeptide to incorporate a translated signal peptide encoding sequence. Generally, signal peptides are proteolytically cleaved from a residual protein as part of the secretory process in which the protein is transported into the host cell periplasm or culture medium.

It is well known in the art that signal peptides facilitate the extracellular discharge of secretory proteins in both prokaryotic and eukaryotic environments. It has been shown that the addition of a heterologous signal peptide to a normally cytosolic protein will result in the extracellular transport of the normally cytosolic protein in *E. coli.* (MacIntyre, *et al*., 1987). It is well known in the art that alternate signal peptide sequences may function with heterologous coding sequences. The recombinant production of such fusion proteins maybe accomplished by the addition of a DNA sequence encoding a signal peptide appropriate to the host organism inserted 5' to, and in reading frame with, the protein coding sequence.

Signal peptides are well known in the art which could be similarly incorporated into the modified M2 polypeptide structure. In the preferred practice of the invention the signal peptide used is a signal peptide native to a secretory protein of the host cell line. Furthermore, the signal sequence may be wholly synthetic. Synthetic "idealized" signal peptides have been shown to function in both prokaryotic and eukaryotic environments. (von Heijne, G.,1990). The principles of signal peptides are similar in both prokaryotic and eukaryotic organisms. Both prokaryotic and eukaryotic signal peptides possess an overall three domain structure and with no precise sequence conservation necessary to preserve function. (von Heijne, G., *supra*). Generally, the presence of basic and/or charged amino acid residues near the amino terminus of the structural protein inhibits secretion. (Yamane, K., *et al*., 1988; Summers, R.G., *et al*., 1989). In order to ensure the efficient cleavage of the signal peptide from the fusion protein construct, it is desirable to maintain the nature of the amino acid sequence at the interface between the signal peptide and the coding sequence of the mature art protein. Conservation of charge and hydrophobicity and the elimination of charged residues immediately downstream of the signal peptide cleavage point are generally important to efficient translocation. However, it is not critical that any one particular amino acid sequence be maintained.

### Eukaryotic Promoters

Preferred promoters controlling transcription in mammalian host cells may be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, *e.g.*, b-actin promoter. The early and late promoters of the SV40 virus are conveniently obtained as an SV40 restriction fragment which also contains the SV40 viral origin of replication. (Fiers, *et al*., 1978). The entire SV40 genome may be obtained from plasmid pBRSV, ATCC 45019. The immediate early promoter of the human cytomegalovirus may be obtained from plasmid pCMBb (ATCC 77177). Of course, promoters from the host cell or related species also are useful herein.

### Eukaryotic Enhancers

Transcription of a DNA encoding modified M2 polypeptides by higher eukaryotes is increased by inserting an enhancer sequence into the vector. Enhancers are cis-acting elements of DNA, usually about 10-300 bp, that act on a promoter to increase its transcription. Enhancers are relatively orientation and position independent having been found 5' (Laimins, L. *et al*., 1981) and 3' (Lusky, M. L., *et al*., 1983) to the transcription unit, within an intron (Banerji, J. L. *et al*., 1983) as well as within the coding sequence itself (Osbome, T. F., *et al*., 1984). Many enhancer sequences are now known from mammalian genes (globin, RSV, SV40, EMC, elastase, albumin, a-fetoprotein and insulin). Typically, however, one will use an enhancer from a eukaryotic cell virus. Examples include the SV40 late enhancer, the cytomegalovirus early promoter enhancer, the polyoma enhancer on the late side of the replication origin, and adenovirus enhancers.

### Eukaryotic Expression Vectors

Expression vectors used in eukaryotic host cells (yeast, fungi, insect, plant, animal, human or nucleated cells from other multicellular organisms) will also contain sequences necessary for the termination of transcription which may affect mRNA expression. These regions are transcribed as polyadenylated segments in the untranslated portion of the mRNA encoding modified M2 polypeptides. The 3' untranslated regions also include transcription termination sites.

### Eukaryotic Selectable Markers

Expression vectors may contain a selection gene, also termed a selectable marker. Examples of suitable selectable markers for mammalian cells are dihydrofolate reductase (DHFR, which may be derived from the BglII/HindIII restriction fragment of pJOD-10 [ATCC 68815]), thymidine kinase (herpes simplex virus thymidine kinase is contained on the BamHI fragment of vP-5 clone [ATCC 2028]) or neomycin (G418) resistance genes (obtainable from pNN414 yeast artificial chromosome vector [ATCC 37682]). When such selectable markers are successfully transferred into a mammalian host cell, the transfected mammalian host cell can survive if placed under selective pressure. There are two widely used distinct categories of selective regimes. The first category is based on a cell's metabolism and the use of a mutant cell line which lacks the ability to grow without a supplemented media. Two examples are: CHO DHFR⁻ cells (ATCC CRL-9096) and mouse LTK- cells (L-M(TK-) ATCC CCL-2.3). These cells lack the ability to grow without the addition of such nutrients as thymidine or hypoxanthine. Because these cells lack certain genes necessary for a complete nucleotide synthesis pathway, they cannot survive unless the missing nutrients are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in non-supplemented media.

The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin (Southern P. and Berg, P., 1982), mycophenolic acid (Mulligan, R. C. and Berg, P. 1980) or hygromycin (Sugden, B. *et al*., 1985). The three examples given above employ bacterial genes under eukaryotic control to convey resistance to the appropriate drug G418 or neomycin (geneticin), xgpt (mycophenolic acid) or hygromycin, respectively.

### Eukaryotic Host Cells

Host cells may be transformed with the expression vectors of this invention and cultured in conventional nutrient media modified as is appropriate for inducing promoters, selecting transformants or amplifying genes. The culture conditions, such as temperature, pH and the like, are those previously used with the host cell selected for expression, and will be apparent to the ordinarily skilled artisan. The techniques of transforming cells with the aforementioned vectors are well known in the art and may be found in such general references as Maniatis, *et al.* (1989). Molecular Cloning: A Laboratory Manual, Cold Spring Harbor Press, Cold Spring Harbor Laboratory, Cold Spring Harbor, New York or Current Protocols in Molecular Biology (1989) and supplements.

Preferred suitable host cells for expressing the vectors of this invention encoding modified M2 polypeptides in higher eukaryotes include: African green monkey kidney line cell line transformed by SV40 (COS-7, A3TCC CRL-1651); transformed human primary embryonal kidney cell line 293,(Graham, F. L. *et al*. 1977); baby hamster kidney cells (BHK-21(C-13), ATCC CCL-10); chinese hamster ovary cells CHO-DHFR- (ATCC CRL-9096), mouse Sertoli cells (TM4, ATCC CRL-1715); African green monkey kidney cells (VERO 76, ATCC CRL-1587); human cervical epitheloid carcinoma cells (HeLa, ATCC CCL-2); canine kidney cells (MDCK, ATCC CCL-34); buffalo rat liver cells (BRL 3A, ATCC CRL-1442); human diploid lung cells (WI-38, ATCC CCL-75); human hepatocellular carcinoma cells (Hep G2, ATCC HB-8065);and mouse mammary tumor cells (MMT 060562, ATCC CCL51).

### Yeast Expression

In addition to prokaryotes, eukaryotic microbes such as yeast cultures may also be used. *Saccharomyces cerevisiae,* or common baker's yeast is the most commonly used eukaryotic microorganism, although a number of other strains are commonly available. For expression in *Saccharomyces,* the plasmid YRp7, for example, (ATCC-40053, Stinchcomb, *et al*., 1979); Kingsman *et al*., 1979); Tschemper *et al*., 1980) is commonly used. This plasmid already contains the trp gene which provides a selectable marker for a mutant strain of yeast lacking the ability to grow in tryptophan, for example ATCC no. 44076 or PEP4-1 (Jones, 1977).

Suitable promoting sequences for use with yeast hosts include the promoters for 3-phosphoglycerate kinase (found on plasmid pAP12BD ATCC 53231 and described in U.S. Patent No. 4,935,350, June 19, 1990) or other glycolytic enzymes such as enolase (found on plasmid pAC1 ATCC 39532), glyceraldehyde-3-phosphate dehydrogenase (derived from plasmid pHcGAPC1 ATCC 57090, 57091), *Zymomonas mobilis* (United States Patent No. 5,000,000 issued March 19, 1991), hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein (contained on plasmid vector pCL28XhoLHBPV ATCC 39475, United States Patent No. 4,840,896), glyceraldehyde 3-phosphate dehydrogenase, and enzymes responsible for maltose and galactose (GAL1 found on plasmid pRY121 ATCC 37658) utilization. Suitable vectors and promoters for use in yeast expression are further described in R. Hitzeman et al., European Patent Publication No. 73,657A. Yeast enhancers such as the UAS Gal from *Saccharomyces cerevisiae* (found in conjuction with the CYC1 promoter on plasmid YEpsec--hI1beta ATCC 67024), also are advantageously used with yeast promoters.

### Expression In Vaccinia

The modified M2 polypeptides may also be expressed in vaccinia virus.

Paoletti, *et al.* (U.S. Patent Nos. 4,722,848 and 5,110,587) describe a general method wherein exogenous DNA sequences are introduced into nonessential regions of the vaccinia virus genome, thereby effecting expression of said exogenous sequences. Paoletti, *et al.* (U.S. Patent No. 5174,993) describes a method for inducing an immunological response in a mammal to a pathogen by incorporation of exogenous DNA sequences derived from the pathogen into avipox virus. The teachings of these patents are hereby incorporated in their entirety be reference. The method of these patents may readily be modified to incorporate modified M2 polypeptide sequences of the present invention.

### Expression By Naked DNA

The modified M2 polypeptides may also be expressed *in vivo* using the "naked DNA" approach as described by Felgner, *et al*. (U.S. Patent No. 5,589,466, 5,703,055, and 5,580,859). This approach entails delivery (typically by injection) of isolated nucleic acids into mammalian tissue, resulting in transient expression of the injected nucleic acids. Transient expression of foreign genes in mammalian tissue invokes an immune response which can be protective. The teachings of this patent are hereby incorporated in their entirety by reference and may readily be modified for use with the modified M2 polypeptide sequences of the present invention.

In another aspect, the invention comprises immunogenic compositions, including vaccines. Such immunogenic compositions comprise a modified M2 protein according to the first aspect of the invention and may be prepared as injectables, as liquid solutions, suspensions or emulsions. The active immunogenic ingredient or ingredients may be mixed with pharmaceutically acceptable excipients which are compatible therewith. Such excipients may include water, saline, dextrose, glycerol, ethanol, and combinations thereof. The immunogenic compositions and vaccines may further contain auxiliary substances, such as wetting or emulsifying agents, pH buffering agents, or adjuvants to enhance the effectiveness thereof. Immunogenic compositions and vaccines may be administered parenterally, by injection subcutaneously or intramuscularly. Alternatively, the immunogenic compositions formed according to the present invention, may be formulated and delivered in a manner to evoke an immune response at mucosal surfaces. Thus, the immunogenic composition may be administered to mucosal surfaces by, for example, the nasal or oral (intragastric) routes. Alternatively, other modes of administration including suppositories and oral formulations may be desirable. For suppositories, binders and carriers may include, for example, polyalkalene glycols or triglycerides. Such suppositories may be formed from mixtures containing the active ingredient(s) in the range of about 0.5 to about 10%, preferably about 1 to 2%. Oral formulations may include normally employed incipients such as, pharmaceutical grades of saccharine, cellulose and magnesium carbonate. These compositions can take the form of solutions, suspensions, tablets, pills, capsules, sustained release formulations or powders and contain about 1 to 95% of the active ingredient(s), preferably about 20 to about 75%.

The immunogenic preparations and vaccines are administered in a manner compatible with the dosage formulation, and in such amount as will be therapeutically effective, protective and immunogenic. The quantity to be administered depends on the subject to be treated, including, for example, the capacity of the individual's immune system to synthesize antibodies, the degree of protection desired, and if needed, to produce a cell-mediated immune response. Precise amounts of active ingredient required to be administered depend on the judgment of the practitioner. However, suitable dosage ranges are readily determinable by one skilled in the art and may be of the order of micrograms of the active ingredient(s) per vaccination. Suitable regimes for initial administration and booster doses are also variable, but may include an initial administration followed by subsequent administrations. The dosage may also depend on the route of administration and will vary according to the size of the host.

The concentration of the active ingredient protein in an immunogenic composition according to the invention is in general about 1 to 95%. A vaccine which contains antigenic material of only one pathogen is a monovalent vaccine. Vaccines which contain antigenic material of several pathogens are combined vaccines and are also contemplated by the present invention. Such combined vaccines contain, for example, material from various pathogens or from various strains of the same pathogen, or from combinations of various pathogens.

Immunogenicity can be significantly improved if the antigens are co-administered with adjuvants, commonly used as 0.05 to 0.1 percent solution in phosphate-buffered saline. Adjuvants enhance the immunogenicity of an antigen but are not necessarily immunogenic themselves. Adjuvants may act by retaining the antigen locally near the site of administration to produce a depot effect facilitating a slow, sustained release of antigen to cells of the immune system. Adjuvants can also attract cells of the immune system to an antigen depot and stimulate such cells to elicit immune responses.

Immunostimulatory agents or adjuvants have been used for many years to improve the host immune responses to, for example, vaccines. Intrinsic adjuvants, such as lipopolysaccharides, normally are the components of the killed or attenuated bacteria used as vaccines. Extrinsic adjuvants are immunomodulators which are typically non-covalently linked to antigens and are formulated to enhance the host immune responses. Thus, adjuvants have been identified that enhance the immune response to antigens delivered parenterally. Some of these adjuvants are toxic, however, and can cause undesirable side-effects, making them unsuitable for use in humans and many animals. Indeed, only aluminum hydroxide and aluminum phosphate (collectively commonly referred to as alum) are routinely used as adjuvants in human and veterinary vaccines. The efficacy of alum in increasing antibody responses to diphtheria and tetanus toxoids is well established and a HBsAg vaccine has been adjuvanted with alum. A wide range of extrinsic adjuvants can provoke potent immune responses to antigens. These include saponins complexed to membrane protein antigens (immune stimulating complexes), pluronic polymers with mineral oil, killed mycobacteria in mineral oil, Freund's complete adjuvant, bacterial products, such as muramyl dipeptide (MDP) and lipopolysaccharide (LPS), as well as lipid A, and liposomes.

To efficiently induce humoral immune responses (HIR) and cell-mediated immunity (CMI), immunogens are often emulsified in adjuvants. Many adjuvants are toxic, inducing granulomas, acute and chronic inflammations (Freund's complete adjuvant, FCA), cytolysis (saponins and pluronic polymers) and pyrogenicity, arthritis and anterior uveitis (LIPS and MDP). Although FCA is an excellent adjuvant and widely used in research, it is not licensed for use in human or veterinary vaccines because of its toxicity.

Desirable characteristics of ideal adjuvants include:
(1) lack of toxicity;
(2) ability to stimulate a long-lasting immune response;
(3) simplicity of manufacture and stability in long-term storage;
(4) ability to elicit both CMI and HIR to antigens administered by various routes, if required;
(5) synergy with other adjuvants;
(6) capability of selectively interacting with populations of antigen presenting cells (APC);
(7) ability to specifically elicit appropriate T_{H}1 or T_{H}2 cell-specific immune responses; and
(8) ability to selectively increase appropriate antibody isotype levels (for example, IgA) against antigens.

US Patent No. 4,855,283 granted to Lockhoff et al on August 8, 1989 which is incorporated herein by reference thereto teaches glycolipid analogues including N-glycosylamides, N-glycosylureas and N-glycosylcarbamates, each of which is substituted in the sugar residue by an amino acid, as immuno-modulators or adjuvants. Thus, Lockhoff et al. (US Patent No. 4,855,283 and ref. 32) reported that N-glycolipid analogs displaying structural similarities to the naturally-occurring glycolipids, such as glycosphingolipids and glycoglycerolipids, are capable of eliciting strong immune responses in both herpes simplex virus vaccine and pseudorabies virus vaccine. Some glycolipids have been synthesized from long-chain-alkylamines and fatty acids that are linked directly with the sugars through the anomeric carbon atom, to mimic the functions of the naturally occurring lipid residues.

U.S. Patent No. 4,258,029 teaches that octadecyl tyrosine hydrochloride (OTH) functioned as an adjuvant when complexed with tetanus toxoid and formalin inactivated type I, II and III poliomyelitis virus vaccine. Also, Nixon-George *et al.* reported that octadecyl esters of aromatic amino acids complexed with a recombinant hepatitis B surface antigen, enhanced the host immune responses against hepatitis B virus.

The choice of adjuvant or combination of adjuvants is entirely within the skills of the ordinarily skilled immunologist. The adjuvants discussed above included adjuvants useful in experimental settings as well as adjuvants of potential human or veterinary application. The influenza A vaccines of the invention can be formulated using any of the aforementioned adjuvants and as such the use of any of the adjuvants in combination or in conjunction with the modified M2 polypeptides of the invention is contemplated by and is thus within the scope of the present invention.

In another aspect, the invention comprises antibodies to M2 which may be produced by a method comprising administering to a subject capable of producing antibodies to M2 a composition according to the invention (which composition comprises a modified M2 protein according to the invention) and collecting antibodies to M2 from the subject.

In another aspect, the invention provides methods for determining current or previous exposure of a subject to influenza virus, the method comprising contacting a sample from the subject with a modified M2 protein according to the first aspect of the invention and detecting the binding of antibodies to the modified M2 protein. The presence of antibodies indicates current or previous exposure of the subject to influenza.

In another aspect, the invention provides the use of a prophylactically effective amount of a modified M2 protein-containing composition according to the invention for the preparation of a pharmaceutical composition for limiting viral influenza A infection in a subject. Prophylactic amounts of the composition can be determined routinely.

In yet another aspect, the invention provides the use of the antibody to M2 according to the invention for the preparation of a pharmaceutical composition for preventing or treating a subject suffering from viral influenza A infection.

The following examples are provided for illustrative purposes only and are not intended, nor should they be construed, as limiting the invention in any manner.

### EXAMPLES

### Example 1

### PCR and plasmid construction

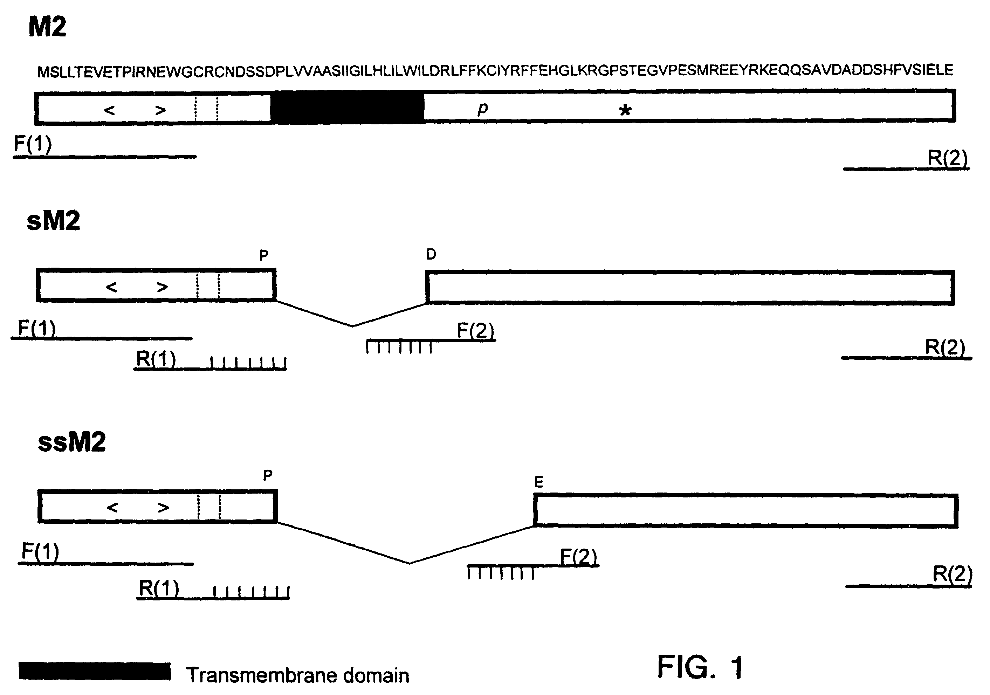

Full length and truncated forms of M2 cDNA were made by PCR from RNA of A/Aichi/2/68 (H3N2) virus. Figure 1 shows schematic diagrams of M2 and the deletion constructs sM2 and ssM2. Each diagram shows a boxed diagram of the M2 structure and the area deleted. Below each are the oligonucleotide primer positions used in constructing the cDNAs. F(1) is forward primer 1, F(2) forward primer 2, R(1) reverse primer 1, (R2) reverse primer 2. (A) The M2 amino acid sequence is represented in three boxes, an extracellular domain, a darkened transmembrane domain, and an intracellular domain. Notations within these boxes include epitopes or post-translational modifications which have been described for M2: (<>) epitope for Mab 14C2; vertical dashed lines are cysteine sulfhydryl linkages; (p) is a palmitoylation site; and (*) is a phosphorylation site. (B) sM2 shows a deletion between Pro(25) and Asp(44). The deletion is performed with primer annealment indicated with vertical lines. ssM2 has a deletion between Pro(25) to Glu(56).

Four oligonucleotide primers were designed to generate cDNA. Forward-1 primer (5'-CCCGAATTCTTATGAGCCTTCTAACCGAGGTCGAAACGCCTATCAGAAACGA-ATGGGGATGC-3') (SEQ ID NO: 1) was specific for the 5' coding region of the M2 gene (nucleotides 1-51) and began with a 5' *EcoR1* restriction site. The reverse-1 primer (5'-GTCTTTGCTTACCCCTACGTCTACGTTGCTAAGTTCACTAGGACCTCCTCCC-3') (SEQ ID NO: 2) [3'-CCCTCCTCCAGGATCACTTGAATCGTTGCATCTGCATCCCC-ATTCGTTTCTG-5'] coded for 3' amplification from nucleotide 75. The forward-2 primer (sM2, 5'-CAAGTGATCCTGGAGGAGGAGATCGTCTCTTCTTCAAATGC-3' (SEQ ID NO: 3); ssM2, 5'-CAAGTGATCCTGGAGGAGGAAAACACGGTCTGAAAAGAGGGCC-3' (SEQ ID NO: 5)) was varied to flank areas chosen for deletion and contained a 5' region homologous to the reverse-1 primer to allow annealing. These primers also coded for three glycine residues inserted in place of the deleted segments. The reverse-2 primer (3'-CTATCAGTAAAGCAGTCGTATCTCGACC-TCATCAGCTGCCC-5') (SEQ ID NO: 4) [5'-CCCGTCGACTACTCCAGCTCTATGCTGACGAAATGAC-TATC-3'] coded for the 3' end of M2 and provided a 3' *Sal1* restriction site. Full-length M2 cDNA was prepared by RT-PCR using forward -1 and reverse -2 primers. For deletion constructs, "5' side" and "3' side" reactions were carried out, annealed, then amplified to produce full length M2 or deletion cDNAs. These were digested with *EcoR1* and *Sal1*, purified by gel electrophoresis and ligated into *EcoR1* and *Sal1* sites of a plasmid vector, pGEX-5Xₐ (Pharmacia, Piscataway, NJ). The construct which has been designated sM2 has a deletion between amino acids 25 and 44 of native M2. The construct designated ssM2 has a deletion between amino acids 25 and 56 of native M2. Plasmids were transformed into competent *E. coli* strain JM109 (Stratagene, La Jolla, CA). Plasmid sequences were verified by automated nucleotide sequence analysis using standard protocols.

### Example 2

### Expression and isolation of fusion protein

The pGEX vector ( Pharmacia, Piscataway, NJ) was chosen to express the constructs, and allows purification of the products with a simple affinity matrix. pGEX is designed to express, under control of the inducible *tac* promoter, glutathione S-transferase (GST; from *Schistosoma japonicum*) as a 29 kDa fusion to the N-terminus of a subcloned sequence (Smith and Johnson, 1988). The fusion protein can be purified from bacterial lysates by affinity chromatography using glutathione sepharose® 4B. The fusion product may also be separated by a site-specific protease, Factor Xa, whose site is immediately downstream of the C-terminus of the GST.

### Soluble fusion proteins

Cells were grown from frozen stocks in overnight cultures of Luria broth (LB) containing 100 µg/ml ampicillin. This culture was then diluted 1:10 the next morning and grown for 1.5 hr at 37°C with vigorous shaking. IPTG (isopropyl β-D-thiogalactoside) was then added to a final concentration of 0.1 mM and incubation continued for 3-4 hrs. To monitor cell growth an aliquot of culture was taken every 0.5 hr after the initial dilution and cell density (A_{600 nm}) was measured over the induction period. Cells were pelleted by centrifugation and resuspended in cold lysis buffer (50 Tris, 100 NaCl, 1 EDTA, pH 8.0). Lysozyme was added to I mg/ml and phenylmethylsulfonylfluoride (PMSF) added to a concentration of 0.5 mM. The suspension was kept on ice for 15 min. Dithiothreitol (DTT) was added to a concentration of 5 mM, and the suspension was lysed by sonication (probe-tip) on ice for 1 min. Triton X-100 was added to a concentration of 1% and the lysate was mixed gently for 0.5 hr. The lysate was then centrifuged at 12,000 x g for 10 min at 4°C. The supernatant was decanted and added to a washed glutathione sepharose® (Pharmacia, Piscataway, NJ) slurry (50% v/v in phosphate-buffered saline (PBS)), with the slurry volume being equal to 0.2% of the original bacterial culture volume. This mixture was gently stirred for 30 min. The sepharose® was then pelleted, the supernatant removed and discarded. The sepharose® was washed a minimum of three times in PBS. Fusion protein was eluted from the sepharose® pellet by addition of elution buffer (50 Tris, 10 reduced glutathione, pH 8.0) using a volume equal to the bed volume of sepharose®. The elution buffer/resin mixture was mixed for 15 min at room temperature, then pelleted at 500 x g for 5 min. The supernatant was harvested and a second elution was performed for residual product.

To enzymatically cleave ssM2 from the GST moiety the fusion product was left bound to sepharose® and treated overnight with 10 µg of Factor Xa (New England Biolabs, Beverly, MA) at 4°C. The sepharose® with bound GST was spun down, and the supernatant, containing the released ssM2, was harvested.

### Insoluble fusion proteins

The insoluble forms of M2 were isolated using the above protocol with the suggested additions of Frangioni and Neel (1993), which include: (1) introduction of 1.5% sarkosyl prior to sonication and (2) raising the concentration of Triton X-100 to 4%. PBS washes of the bound glutathione sepharose® and the elution buffer contained 0.1% Triton X-100.

### Example 3

### Electrophoresis and western blotting

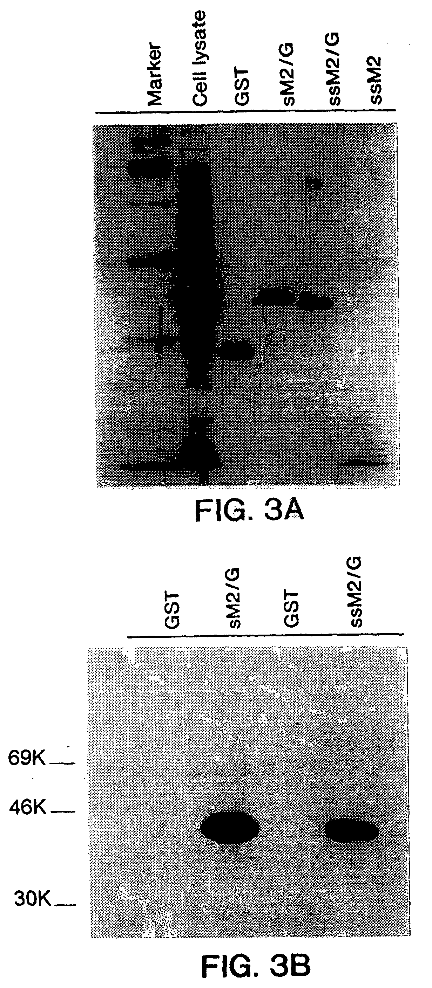

Expressed proteins were analyzed for size and purity on an SDS-12%- polyacrylamide gel, followed by staining with Coomassie brilliant blue R-250. Figure 3 (A) shows an SDS-PAGE gel of recombinant proteins. Lanes are: (1) molecular weight markers (2) a sample from the crude bacterial lysate of an induced sM2/G culture (3) a purified sample of GST protein (4) sM2/G protein (6) ssM2 protein which is isolated by cleaving ssM2/G with factor Xa protease. Molecular weights were compared to low molecular weight Rainbow Markers (Amersham International, Arlington Heights, IL.). For immunoblotting, gels were transferred to Immobilon-P membrane (Millipore, Bedford, MA) using a semi-dry transblot apparatus (Bio-Rad, Richmond, CA). Membranes were immunoblotted with a 1:5000 dilution of ascitic fluid containing the M2-specific antibody 14C2, followed by labeling with the ECL system (Amersham International, Arlington Heights, IL.) and exposure to X-ray film. Figure 3 (B) shows a Western blot of GST, sM2/G and ssM2/G using 14C2 as the primary antibody.

### Example 4

### Animal vaccination and challenge

Fusion proteins or control GST protein were added to equal volumes of PBS and incomplete Freund's adjuvant. A volume of 0.2 ml, containing 10 µg of protein was injected intraperitoneally (i.p.) into female BALB/c mice, aged 6-12 weeks. Boosts were given after 3 and 6 weeks for a total of 3 inoculations. Animals were bled from the orbital plexus at weeks 6 and 9 and individual sera were tested for antibodies which would react with a synthetic peptide composed of the first 17 amino acids of M2 (peptide PM₂-1, Slepushkin *et al.,* 1995). Antibody binding was detected on peptide-coated ELISA plates by adding horseradish peroxidase-conjugated anti-mouse Ig and *o*-phenylenediaminehydrochloride and hydrogen peroxide as colorimetric substrates. Titers are expressed as the highest dilution which yielded an optical density (OD)₄₉₀ two times higher than a similarly diluted control sera.

Following inoculations, mice were subjected to sub-lethal challenge by heterologous influenza A virus. Mice were anesthetized with CO₂ and were infected intranasally (i.n.) with 100 mouse infectious doses (MID)₅₀ of MA A/Ann Arbor/6/60 (H2N2) virus or A/Taiwan/1/86 (H1N1) [equivalent to5.3 x10⁶ and 1.3 x 10⁵ egg infectious doses (EID)₅₀ respectively] in a volume of 50 µl of PBS. Mice were euthanized seven days after challenge. Lung homogenates were prepared and titrated into embryonated eggs for virus infectivity. Statistical significance of the data was determined using the Fisher exact test or Student's *t* test.

### Example 5

### Expression of recombinant M2 and M2 transmembrane deletants

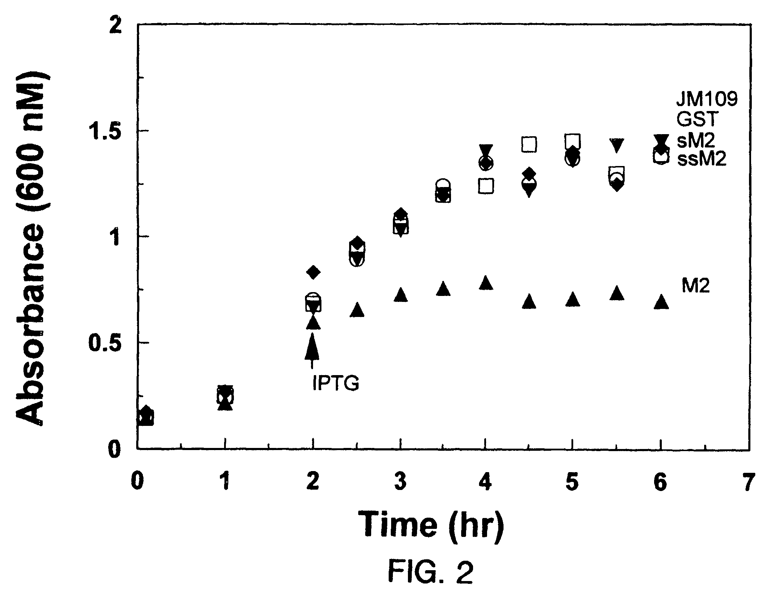

The effects of expression of GST-fusion M2/G, sM2/G, ssM2/G, and GST on cell viability was tested at various times following induction of *E. coli* JM109 cells containing the respective pGEX constructs (Fig.2). Overnight cultures were diluted 1:100, reamplified for 2 hrs, and then induced with IPTG. At the time of induction the density of the M2/G culture was consistently lower than other cultures, presumably due to basal expression of the protein. The density of cells expressing full-length M2/G rose only marginally after induction, consistent with the reported lytic properties of M2 when expressed in *E coli* (Guinea and Carrasco, 1994). Addition of 5 µM amantadine to the culture media did not accelerate this growth pattern, and little, if any, M2/G fusion protein was obtainable from these cultures. In comparison, cultures of sM2/G and ssM2/G maintained a robust pattern of growth comparable to that of the control culture and the GST control protein.

The first deletion construct, which removes the transmembrane domain of M2 (sM2/G, residues 26-43), although expressing well, yielded no purified fusion protein with a standard lysis protocol, suggesting that it remained insoluble or in aggregated form. Adopting the sarkosyl protocol produced a modest yield of ∼3 mg of fusion protein/L of bacterial culture, as determined by Bradford protein assay. Further deletion, from residue 26 to 55 (ssM2/G), was found to substantially improve fusion protein yield without the sarkosyl procedure, suggesting a soluble product. Residues 44-55 of mature M2 are all hydrophobic and contribute to a positive hydrophobic index for M2 in Kyte-Doolittle analysis (Lamb *et al*., 1985). Values of up to 15 mg/L of culture are routinely achieved, with the purity of the fusion proteins in either preparation being >90%.

### Example 6

### SDS-PAGE and Western Blot

GST and fusion proteins were electrophoresed on polyacrylamide gels and either stained with Coomassie blue or prepared for immunoblotting. In Fig. 3 (A) a Coomassie stained gel is shown. A total protein sample from induced cells taken in the initial phase of the lysate is included (lane 2). GST (lane 3), from an induction of pGEX without M2 insert, is observed as a 29 kDa protein. Fusion proteins, sM2/G (lane 4) and ssM2/G (lane 5) are found at approximately 43 kDa. Panel (B) shows proteins run on a gel simultaneously with (A) , transferred to Immobilon-P membrane, and immunoblotted with M2-specific antibody 14C2. Proteins with an approximate weight of 43 kDa reacted with 14C2. GST is not visualized in the blot. Together, these results suggest that the 14C2 antibody epitope of the M2 deletion proteins is not obstructed by the fusion construction and that 14C2 is binding exclusively to the M2 domain.

### Example 7

### Immunogenic and protective properties of recombinant fusion proteins

The immunogenicity and protective efficacy of several M2 constructs were tested by vaccinating groups of BALB/c mice with sM2/G, ssM2/G, and enzymatically isolated ssM2 recombinant proteins. GST peptide was administered as a control. Results are shown in Table 1.

**Table 1**

| *Virus challenge of mice vaccinated with M2 constructs* | | | | |
|---|---|---|---|---|
| **Expt.** | **Vaccine** | **n** | **Serum antibody titer** | **Lung virus titer** |
| 1 | GST | 7 | < 50 | 6.5±0.6 (AA/60) |
| 2 | GST | 5 | < 50 | 6.6±0.9 (AA/60) |
| 3 | PBS | 5 | < 50 | 6.8±0.4 (AA/60) |
| 1 | sM2/G | 7 | 152,054 | 2.6±0.6 (AA/60) |
| 2 | sM2/G | 5 | 19,390 | 2.2±1.5 (AA/60) |
| 2 | ssM2/G | 5 | 11,138 | 2.9±1.4 (AA/60) |
| 3 | ssM2 | 7 | 123,838 | 3.2±0.7 (AA/60) |
| 4 | PBS | 4 | < 50 | 7.4±0.7 (AA/60) |
| 4 | GST | 5 | < 50 | 6.4±0.6 (AA/60) |
| 4 | PBS | 3 | < 50 | 7.0±0.6 (TW/86) |
| 4 | GST | 6 | < 50 | 7.0±1.0 (TW/86) |
| 4 | sM2/G | 5 | 131,825 | 3.0±0.8 (AA/60) |
| 4 | sM2/G | 6 | 131,825 | 3.7±0.5 (TW/86) |
| 4 | ssM2 | 5 | 1,202,264 | 3.2±1.2 (AA/60) |
| 4 | ssM2 | 6 | 1,202,264 | 3.6±0.5 (TW/86) |
| Challenge viruses were mouse adapted A/AA/6/60 (H2N2) and A/Taiwan/1/86 (H1N1). | | | | |
| All vaccinations were 3 x 10 µg of antigen, in Incomplete Freund's adjuvant. | | | | |
| ^{a} titers are expressed as the highest dilution of sera having a mean (OD₄₉₀ greater than the mean plus two standard deviations of similarly diluted control sera. | | | | |
| ^{b} Mean Log₁₀EID₅₀/ml ± SD. Values for all virus titer reductions were significantly lower than control groups by Students *t*-test (p<0.001). | | | | |

Groups of 5 to 7 mice were vaccinated as described above and analyzed for serum antibody which could recognize a synthetic peptide designed to mimic the extracellular domain of M2. Sera from mice vaccinated with GST control peptide showed no detectable (<50) antibody titer in any samples prior to challenge. However, groups vaccinated with sM2/G, ssM2/G, and ssM2 proteins showed elevated titers after two inoculations and a third inoculation boosted the mean serum antibody titers for each group substantially. Surprisingly, deletion of the transmembrane region and additional hydrophobic residues of M2 does not appear to alter the immunological properties of the variant polypeptide, making such variant polypeptides suitable candidates for vaccines.

For virus challenge, mice vaccinated with either GST, sM2/G, ssM2/G, or ssM2 were challenged with heterologous MA A/Ann Arbor/6/60 (H2N2) or A/Taiwan/1/86 (H1N1) virus 4 weeks after their final boost. After 7 days the mice were euthanized, and lungs were harvested. Lung virus titers for the sM2/G and ssM2/G fusion protein groups, and isolated ssM2, were over 1000-fold lower than the PBS or GST protein control groups (Table 2). No significant difference was found between the sM2/G, ssM2/G, and ssM2 protective effect. This trend demonstrates a potential of the altered M2 peptides to protect against virus challenge, as has been shown for full-length baculovirus M2. Immunogenic compositions, suitable to be used as vaccines, may be prepared from immunogenic M2 as disclosed herein. Preferably, the antigenic material is extensively dialyzed to remove undesired small molecular weight molecules and/or lyophilized for more ready formulation into a desired vehicle. The immunogenic composition elicits an immune response which produces antibodies, including M2 antibodies which may inhibit viral replication, and also influenza A virus-specific cell-mediated immune responses.

The protocols and discussion provided above are sufficient to enable skilled workers in the field to reproduce the claimed inventions. The present inventors supplement the disclosure by listing scientific publications pertinent to the protocols and materials used to convey the present invention.

### REFERENCES

Banerji, J. L. *et al*., (1983) Cell 33:729.

Black, R. A., Rota, P. A., Gorodkova, N., Cramer, A., Klenk, H. D. & Kendal, A. P. (1993) Production of the M2 protein of influenza A virus in insect cells is enhanced in the presence of amantadine. *Journal of General Virology* **74,** 1673-1677.

Black, R. A., Rota, P. A., Gorodkova, N., Klenk, H. D. & Kendal, A. P. (1993) Antibody response of M2 protein of influenza A virus expressed in insect cells. *Journal of General. Virology* **74,** 143-146.

Bolivar, *et al*., (1977) Gene 2: 95.

Brophy, P. M. & Pritchard, D. I. (1994) Parasitic helminth glutathione s-transferases: an update on their potential as targets for immuno- and chemotherapy. *Experimental Parasitology* **79**, 89-96.

Brown, *et al.* (1979). Methods in Enzymology, Academic Press, N.Y., Vol. 68, pgs. 109-151.

Carter P., (1990) Site Specific Proteolysis of Fusion Proteins, Ch. 13 in Protein Purification: From Molecular Mechanisms to Large Scale Processes, American Chemical Soc., Washington, D.C.

Chang, *et al.,* (1978) Nature, 275:615.

Ciampor, F., Thompson, C. A., Grambas, S. & Hay, A. J. (1992*b*) Regulation of pH by the M2 protein of influenza A viruses. *Virus Research* **22,** 247-258.

Cox, N. J. & Bender, C. A. (1995) The molecular epidemiology of influenza viruses. *Seminars in Virology* **6**, 359-370.

Epstein, S. L., Misplon, J. A., Lawson, C. M., Subbarao, E. K., Connors, M. & Murphy, B. R. (1993) β 2-microglobulin-deficient mice can be protected against influenza A infection by vaccination with vaccinia-influenza recombinants expressing hemagglutinin and neuraminidase. *Journal of Immunology* **150**, 5484-5493.

Fiers, *et al*., (1978) Nature, 273:113

Fikrig, E., Barthold, S. W., Kantor, F. S. & Flavell, R. A. (1990) Protection of mice against the lyme disease agent by immunizing with recombinant OspA. *Science* **250,** 553-555.

Frangioni, J. V. & Neel, B. G. (1993) Solubilization and purification of enzymatically active glutathione s-transferase pGEX fusion proteins. *Analytical Biochemistry.* **210,** 179-187.

Goeddel, *et al.,* (1979) Nature 281:544.

Graham, F. L. *et al.* (1977) J. Gen Virol. 36:59-72, Virology 77:319-329, Virology 86:10-21.

Grambas, S. & Hay, A. J. (1992) Maturation of influenza A virus hemagglutinin - estimates of the pH encountered during transport and its regulation by the M2 protein. *Virology* **190,** 11-18.

Guinea, R. & Carrasco, L. (1994) Influenza virus M2 protein modifies membrane permeability in *E. coli* cells. *FEBS Letters* **343,** 242-246.

Holsinger, L. J., Shaughnessy, M. A., Micko, A., Pinto, L. H. & Lamb, R. A. (1995) Analysis of the posttranslational modifications of the influenza virus M2 protein. *Journal of Virology* **69**, 1219-1225.

Hughey, P. G., Roberts, P. C., Holsinger, L. J., Zebedee, S. L., Lamb, R. A. & Compans, R. W. (1995) Effects of antibody to the influenza A virus M2 protein on M2 surface expression and virus assembly. *Virology* **212**, 411-421.

Ito, T., Gorman, O. T., Kawaoka, Y., Bean, W. J. & Webster, R. G. (1991) Evolutionary analysis of the influenza A virus M gene with comparison of the M1 and M2 proteins. *Journal of Virology* **65**, 5491-5498.

Jacob, C. O., Leitner, M., Zamir, A., Salomon, D. & Arnon, R. (1985) Priming immunization against cholera toxin and E. Coli heat-labile toxin by a cholera toxin short peptide-beta-galactosidase hybrid synthesized in E. Coli. *Embo Journal* **4**, 3339-3343.

Jakeman, K. J., Smith, H. & Sweet, C. (1989) Mechanism of immunity to influenza: maternal and passive neonatal protection following immunization of adult ferrets with a live vaccinia-influenza virus hemagglutinin recombinant but not with recombinants containing other influenza virus proteins. *Journal of General Virology* **70**, 1523-1531.

Johnson, K. S., Harrison, G. B. L., Lightowlers, M. W., O'Hoy, K. L., Cougle, W. G., Dempster, R. P., Lawrence, S. B., Vinton, J. G., Heath, D. D. & Rickard, M. D. (1989) Vaccination against ovine cysticercosis using a defined recombinant antigen. *Nature* **338,** 585-587.

Jones (1977), Genetics 85:12.

Katz, J.M., Black, R.A., Rowe, T., Slepushkin, V.A. and Cox, N.J. (1996). Immune mechanisms of protection induced by vaccination of Balb/c mice with influenza A virus M2 protein, pp. 837-43, In: Options for the Control of Influenza III, (L.E. Brown, A.W. Hampson,

R.G. Webster, eds.), Elsevier Science, Amsterdam.

Kingsman, *et al*., (1979) Gene 7:141.

Kleid, D. G., Yansura, D., Small, B., Dowbenko, D., Moore, D. M., Grubman, M. J., McKercher, P. D., Morgan, D. O., Robertson, B. H. & Bachrach H. L. (1981) Cloned viral protein vaccine for foot-and-mouth disease: responses in cattle and swine. *Science* **214,** 1125-1129.

Laimins, L. *et al*., (1981) PNAS 78:993.

Lamb, R. A., Zebedee, S. L. & Richardson, C. D. (1985) Influenza virus M2 protein is an integral membrane protein expressed on the infected cell surface. *Cell* **40**, 627-633.

Ling, I. T., Ogun, S. A. & Holder, A. A. (1994) Immunization against malaria with a recombinant protein. *Parasite Immunology* **16**, 63-67.

Lusky, M. L., *et al.,* (1983) Mol. Cell Bio. 3:1108.

MacIntyre, *et al*.,(1987) J.Biol.Chem. 262:8416-8422.

Maniatis, *et al.* (1989). Molecular Cloning: A Laboratory Manual, Cold Spring Harbor Press, Cold Spring Harbor Laboratory, Cold Spring Harbor, New York.

Mulligan, R. C. and Berg, P. (1980) Science 209:1422.

Nixon-George *et al*.

Osborne, T. F., *et al*., (1984) Mol. Cell Bio. 4:1293.

Shimbo, K., Brassard, D. L., Lamb, R. A. & Pinto, L. H. (1996) Ion selectivity and activation of the M2 Ion channel of influenza virus. *Biophysical Journal* **70,** 1335-1346.

Slepushkin, V. A., Katz, J. M., Black, R. A., Gamble, W. C., Rota, P. A. & Cox, N. J. (1995) Protection of mice against influenza A virus by vaccination with baculovirus expressed M2 protein. *Vaccine* **15,** 1399-1402.

Smith, D. B. & Johnson, K. S. (1988) Single-step purification of polypeptides expressed in *Escherichia coli* as fusions with glutathione *s*-transferase. *Gene* **67,** 31-40.

Southern P. and Berg, P., (1982) J. Molec. Appl. Genet. 1: 327.

Stinchcomb, *et al.,* (1979) Nature 282:39.

Srivastava, A. K., Morita, K., Matsuo, S., Tanaka, M. & Igarashi, A. (1991) Japanese encephalitis virus fusion protein expressed in *Escherichia coli* confers protective immunity in mice. *Microbiological Immunology* **35,** 863-870.

Srivastava, A. K., Putnak, J. R., Warren, R. L. & Hoke, C. H. (1995) Mice immunized with a dengue type 2 virus E and NS1 fusion protein made in *Escherichia coli* are protected against lethal dengue virus infection. *Vaccine* **13,** 1251-1258.

Sugden, B. *et al*., (1985) Mol Cell. Biol. 5:410-413

Sugrue, R. J., Bahadur, G., Zambon, M. C., Hall-Smith, M., Douglas, A. R. & Hay, A. J. (1990) Specific structural alteration of the influenza hemagglutinin by amantadine. *Embo Journal* **9**, 3469-3476.

Summers, R.G., *et al*. (1989) J.Biol.Chem. 264:20082-20088.

Tosteson, M. T., Pinto, L. H., Holsinger, L. J. & Lamb, R. A. (1994) Reconstitution of the influenza virus M2 channel in lipid bilayers. *Journal of Membrane Biology* **142,** 117-126.

Treanor, J. J., Tiemey, E. L., Zebedee, S. L., Lamb, R. A. & Murphy, B. R. (1990) Passively transferred monoclonal antibody to the M2 protein inhibits influenza A virus replication in mice. *Journal of Virology* **64**, 1375-1377.

Tschemper *et al*., (1980) Gene 10:157.

von Heijne, G. (1990) J. Membrane Biol. 115: 195-201

Wilson, I. A. & Cox, N. J. (1990) Structural basis of immune recognition of influenza hemagglutinin. *Annual Reviews in Immunology* **8,** 737-771.

Yamane, *K., et al*. (1988) J.Biol.Chem. 263:19690-19696.

Zebedee, S. L. & Lamb, R. A. (1988) Influenza A virus M2 protein: monoclonal antibody restriction of virus growth and detection of M2 in virions. *Journal of Virology* **62,** 2762-2772.

## Claims

1. A modified influenza A viral surface polypeptide M2 with reduced hydrophobicity and enhanced recombinant expression relative to a native M2, the modified M2 polypeptide comprising a sequence of amino acids identical to a native M2 protein in which the transmembrane region and from 0 to 12 amino acid residues adjacent to the transmembrane region on the C-terminal side have been deleted.

2. The modified M2 polypeptide of claim 1, wherein the transmembrane region and none of the adjacent residues on the C-terminus side of the transmembrane region have been deleted.

3. The modified M2 polypeptide of claim 1, wherein the transmembrane region and the adjacent 12 amine acids on the C-terminal side of the transmembrane region have been deleted.

4. The modified M2 polypeptide of claim 1, wherein the native M2 protein is from the A/Aichi/2/68 (H3N2) virus.

5. The modified M2 polypeptide of claim 4, wherein amino acids 26-43 have been deleted.

6. The modified M2 polypeptide of claim 4, wherein amino acids 26-55 have been deleted.

7. The modified M2 polypeptide of any one of claims 1 to 6, wherein the deleted amino acid residues are replaced with one or more neutral or hydrophilic amino acid residues, provided that the total number of amino acid residues in the modified M2 polypeptide is less than or equal to the number in the native M2 polypeptide.

8. The modified M2 polypeptide of claim 7, wherein all of the deleted amino acids are replaced with from one to six glycine residues.

9. A modified influenza A viral surface polypeptide M2 with reduced hydrophobicity and enhanced recombinant expression relative to a native M2, the modified M2 polypeptide comprising a sequence of amino acids identical to a native M2 protein in which all of the amino acid residues of the transmembrane region and from 0 to 12 amino acid residues adjacent to the transmembrane region on the C-terminal side are replaced with neutral or hydrophilic amino acid residues.

10. The modified M2 polypeptide of claim 9, wherein all of the amino acid residues of the transmembrane region and from one to twelve amino acids adjacent to the transmembrane region on the C-terminal side have been substituted with neutral or hydrophilic residues.

11. The modified M2 polypeptide of claim 9 or 10, wherein the native M2 protein is from the A/Aichi/2/68 (H3N2) virus.

12. A modified M2 polypeptide fusion protein comprising a modified M2 polypeptide according to and one of claims 1 to 11.

13. A DNA molecule comprising a sequence of nucleotides encoding a modified M2 polypeptide according to any one of claims 1 to 11.

14. A vector capable of expressing a modified M2 polypeptide, the vector comprising the DNA molecule of claim 13.

15. A host cell capable of expressing a modified M2 polypeptide, the host cell comprising a vector according to claim 14.

16. The host cell according to claim 15, wherein the host is a prokaryote.

17. The host cell according to claim 15, wherein the prokaryote is *E. coli.*

18. A composition comprising a modified M2 polypeptide of any one of claims 1 to 11 and a pharmaceutically acceptable carrier.

19. An antibody specific to a modified M2 polypeptide of any one of claims 1 to 11.

20. The use of a prophylactic or viral load-reducing amount of an antibody according to claim 19 for the preparation of a pharmaceutical composition for preventing or treating a subject suffering from viral influenza A infection.

21. A method for determining current or previous exposure of a subject to influenza virus, the method comprising contacting a sample from the subject with a modified M2 protein according to any one of claims 1 to 11 and detecting the binding of antibodies to the modified M2 protein.

## Patentansprüche

1. Modifiziertes virales Oberflächenpolypeptid M2 von Influenza A mit verminderter Hydrophobizität und verbesserter rekombinanter Expression im Verhältnis zu einem nativen M2, wobei das modifizierte M2-Polypeptid eine Sequenz von Aminosäuren umfasst, die identisch zu der eines nativen M2-Proteins ist, in der die Transmembranregion und 0 bis 12 Aminosäurereste, die zu der Transmembranregion an der C-terminalen Seite benachbart sind, deletiert wurden.

2. Modifiziertes M2-Polypeptid nach Anspruch 1, wobei die Transmembranregion und keine der benachbarten Reste an der C-Terminusseite der Transmembranregion deletiert wurden.

3. Modifiziertes M2-Polypeptid nach Anspruch 1, wobei die Transmembranregion und die 12 benachbarten Aminosäuren auf der C-terminalen Seite der Transmembranregion deletiert wurden.

4. Modifiziertes M2-Polypeptid nach Anspruch 1, wobei das native M2-Protein von dem A/Aichi/2/68 (H3N2)-Virus abstammt.

5. Modifiziertes M2-Polypeptid nach Anspruch 4, wobei die Aminosäuren 26 bis 43 deletiert wurden.

6. Modifiziertes M2-Polypeptid nach Anspruch 4, wobei die Aminosäuren 26 bis 55 deletiert wurden.

7. Modifiziertes M2-Polypeptid nach einem der Ansprüche 1 bis 6, wobei die deletierten Aminosäurereste mit einem oder mehreren neutralen oder hydrophilen Aminosäureresten ersetzt sind, mit der Maßgabe, dass die Gesamtanzahl an Aminosäureresten in dem modifizierten M2-Polypeptid weniger oder gleich der Anzahl in dem nativen M2-Polypeptid ist.

8. Modifiziertes M2-Polypeptid nach Anspruch 7, wobei alle deletierten Aminosäuren mit 1 bis 6 Glycinresten ersetzt sind.

9. Modifiziertes virales Oberflächenpolypeptid M2 von Influenza A mit verminderter Hydrophobizität und verbesserter rekombinanter Expression im Verhältnis zu einem nativen M2, wobei das modifizierte M2-Polypeptid eine Sequenz von Aminosäuren umfasst, die identisch zu der eines nativen M2-Proteins ist, in der alle Aminosäurereste der Transmembranregion und 0 bis 12 Aminosäurereste, die zu der Transmembranregion auf der C-terminalen Seite benachbart sind, mit neutralen oder hydrophilen Aminosäureresten ersetzt sind.

10. Modifiziertes M2-Polypeptid nach Anspruch 9, wobei alle Aminosäurereste der Transmembranregion und 1 bis 12 Aminosäuren, die zu der Transmembranregion auf der C-terminalen Seite benachbart sind, mit neutralen oder hydrophilen Resten substituiert wurden.

11. Modifiziertes M2-Polypeptid nach Anspruch 9 oder 10, wobei das native M2-Protein von dem A/Aichi/2/68 (H3N2)-Virus abstammt.

12. Fusionsprotein eines modifizierten M2-Polypeptids, das ein modifiziertes M2-Polypeptid gemäß einem der Ansprüche 1 bis 11 umfasst.

13. DNA-Molekül, das eine Sequenz von Nukleotiden umfasst, die für ein modifiziertes M2-Polypeptid gemäß einem der Ansprüche 1 bis 11 kodiert.

14. Vektor, der zum Exprimieren eines modifizierten M2-Polypeptids fähig ist, wobei der Vektor das DNA-Molekül nach Anspruch 13 umfasst.

15. Wirtszelle, die zum Exprimieren eines modifizierten M2-Polypeptids fähig ist, wobei die Wirtszelle einen Vektor gemäß Anspruch 14 umfasst.

16. Wirtszelle gemäß Anspruch 15, wobei der Wirt ein Prokaryot ist.

17. Wirtszelle gemäß Anspruch 15, wobei der Prokaryot *E*. *coli* ist.

18. Zusammensetzung, die ein modifiziertes M2-Polypeptid nach einem der Ansprüche 1 bis 11 und einen pharmazeutisch verträglichen Träger umfasst.

19. Antikörper, der für ein modifiziertes M2-Polypeptid nach einem der Ansprüche 1 bis 11 spezifisch ist.

20. Verwendung einer prophylaktischen oder Virus-Last-vermindernden Menge eines Antikörpers gemäß Anspruch 19 zur Herstellung einer pharmazeutischen Zusammensetzung zur Prophylaxe oder Behandlung in einem Patienten, der an einer Influenza A-Virusinfektion leidet.

21. Verfahren zum Bestimmen einer gegenwärtigen oder früheren Aussetzung eines Patienten gegenüber Influenza-Virus, wobei das Verfahren ein In-Kontakt-Bringen einer Probe des Patienten mit einem modifizierten M2-Protein nach einem der Ansprüche 1 bis 11 und Nachweisen der Bindung von Antikörpern an das modifizierte M2-Protein umfasst.

## Revendications

1. Polypeptide de surface du virus grippal A, M2 modifié ayant une hydrophobicité réduite et une expression recombinante accrue par rapport au M2 natif, le polypeptide M2 modifié comprenant une séquence d'acides aminés identique à une protéine M2 native dans laquelle la région transmembranaire et de 0 à 12 résidus d'acides aminés adjacents à la région transmembranaire sur le côté C-terminal ont été délétés.

2. Polypeptide M2 modifié selon la revendication 1, dans lequel la région transmembranaire et aucun des résidus adjacents sur le côté C-terminal de la région transmembranaire ont été délétés.

3. Polypeptide M2 modifié selon la revendication 1, dans lequel la région transmembranaire et les 12 acides aminés adjacents sur le côté C-terminal de la région transmembranaire ont été délétés.

4. Polypeptide M2 modifié selon la revendication 1, dans lequel la protéine M2 native est issue du virus A/Aichi/2/68 (H3N2).

5. Polypeptide M2 modifié selon la revendication 4, dans lequel les acides aminés 26-43 ont été délétés.

6. Polypeptide M2 modifié selon la revendication 4, dans lequel les acides aminés 26-55 ont été délétés.

7. Polypeptide M2 modifié selon l'une quelconque des revendications 1 à 6, dans lequel les résidus d'acides aminés délétés sont remplacés par un ou plusieurs résidus d'acides aminés neutres ou hydrophiles, à la condition que le nombre total de résidus d'acides aminés dans le polypeptide M2 modifié soit inférieur ou égal au nombre dans le polypeptide M2 natif.

8. Polypeptide M2 modifié selon la revendication 7, dans lequel la totalité des acides aminés délétés sont remplacés par un à six résidus glycine.

9. Polypeptide de surface du virus grippal A, M2 modifié ayant une hydrophobicité réduite et une expression recombinante accrue par rapport au M2 natif, le polypeptide M2 modifié comprenant une séquence d'acides aminés identique à une protéine M2 native dans laquelle la totalité des résidus d'acides aminés de la région transmembranaire et de 0 à 12 rédisus d'acides aminés adjacents à la région transmembranaire sur le côté C-terminal sont remplacés par des résidus d'acides aminés neutres ou hydrophiles.

10. Polypeptide M2 modifié selon la revendication 9, dans lequel la totalité des résidus d'acides aminés de la région transmembranaire et de un à douze acides aminés adjacents à la région transmembranaire sur le côté C-terminal ont été substitués par des résidus neutres ou hydrophiles.

11. Polypeptide M2 modifié selon la revendication 9 ou 10, dans lequel la protéine M2 native est issue du virus A/Aichi/2/68 (H3N2).

12. Protéine de fusion de polypeptide M2 modifié comprenant un polypeptide M2 modifié selon l'une quelconque des revendications 1 à 11.

13. Molécule d'ADN comprenant une séquence de nucléotides codant pour un polypeptide M2 modifié selon l'une quelconque des revendications 1 à 11.

14. Vecteur capable d'exprimer un polypeptide M2 modifié, le vecteur comprenant la molécule d'ADN selon la revendication 13.

15. Cellule hôte capable d'exprimer un polypeptide M2 modifié, la cellule hôte comprenant un vecteur selon la revendication 14.

16. Cellule hôte selon la revendication 15, dans laquelle l'hôte est un procaryote.

17. Cellule hôte selon la revendication 15, dans laquelle le procaryote est *E. coli*.

18. Composition comprenant un polypeptide M2 modifié selon l'une quelconque des revendications 1 à 11 et un support pharmaceutiquement acceptable.

19. Anticorps spécifique pour un polypeptide M2 modifié selon l'une quelconque des revendications 1 à 11.

20. Utilisation d'une quantité prophylactique ou réduisant la charge virale d'un anticorps selon la revendication 19 pour la préparation d'une composition pharmaceutique pour la prévention ou le traitement d'un sujet souffrant d'une infection par virus grippal A.

21. Procédé pour la détermination de l'exposition actuelle ou précédente d'un sujet au virus de la grippe, le procédé comprenant la mise en contact d'un échantillon issu du sujet avec une protéine M2 modifiée selon l'une quelconque des revendications 1 à 11, et la détection de la liaison d'anticorps à la protéine M2 modifiée.


### SEQUENCE LISTING

<110> Frace, Michael
   Klimov, Alexander
   Katz, Jaquelline
   Centers for Disease Control and Prevention
<120> PREPARATION AND USE OF RECOMBINANANT INFLUENZA A VIRUS M2 CONSTRUCTS AND VACCINES
<130> Modified M2 Protein
<140>
   <141>
<150> U.S. 08/906,930
   <151> 1997-08-06
<160> 5
<170> PatentIn Ver. 2.0
<210> 1
   <211> 62
   <212> DNA
   <213> Artificial Sequence
<220>
   <223> Description of Artificial Sequence: Forward-1 primer specific for the 5' coding region of the M2 gene
<400> 1
   cccgaattct tatgagcctt ctaaccgagg tcgaaacgcc tatcagaaac gaatggggat 60
   gc                                                                62
<210> 2
   <211> 52
   <212> DNA
   <213> Artificial Sequence
<220>
   <223> Description of Artificial Sequence: reverse-1 primer coding for 3' amplification from nucleotide 75 of M2 protein.
<400> 2
   gtctttgctt acccctacgt ctacgttgct aagttcacta ggacctcctc cc         52
<210> 3
   <211> 41
   <212> DNA
   <213> Artificial Sequence
<220>
   <223> Description of Artificial Sequence: Forward-2 primer for sM2 containing a 5' region homologous to the reverse-1 primer
<400> 3
   caagtgatcc tggaggagga gatcgtctct tcttcaaatg c                     41
<210> 4
   <211> 41
   <212> DNA
   <213> Artificial Sequence
<220>
   <223> Description of Artificial Sequence: reverse-2 primer coding for the 3' end of M2.
<400> 4
   ctatcagtaa agcagtcgta tctcgacctc atcagctgcc c                     41
<210> 5
   <211> 43
   <212> DNA
   <213> Artificial Sequence
<220>
   <223> Description of Artificial Sequence: Forward-2 primer for ssM2 containing a 5' region homologous to the reverse-1 primer
<400> 5
   caagtgatcc tggaggagga aaacacggtc tgaaaagagg gcc                43